# EUROPÄISCHE PATENTSCHRIFT

(11) **EP 1 638 531 B1**
(45) Veröffentlichungstag und Bekanntmachung des Hinweises auf die Patenterteilung: **13.02.2013**
(21) Anmeldenummer: 04729885.6
(22) Anmeldetag: 28.04.2004
(51) Int. Cl.: A61K 9/16, A61K 9/51, A61K 39/00

(54) **ANTIGENHALTIGE MIKROSPHÄREN ZUR ALLERGIETHERAPIE**
ANTIGEN-CONTAINING MICROSPHERES FOR THE TREATMENT OF ALLERGIES
MICROSPHERES CONTENANT DES ANTIGENES POUR LE TRAITEMENT D'ALLERGIES

(30) Priorität: 27.06.2003 DE 10329087
(43) Veröffentlichungstag der Anmeldung: 29.03.2006
(73) Patentinhaber: Biomedical International R + D GmbH, 1220 Wien (AT)
(72) Erfinder: JENSEN-JAROLIM, Erika, A-1210 Wien (AT); WALTER, Franziska, A-1090 Wien (AT); GABOR, Franz, A-1090 Wien (AT)
(74) Vertreter: Kador & Partner
(86) Internationale Anmeldenummer: PCT/EP2004/004480
(87) Internationale Veröffentlichungsnummer: WO 2005/000274

(56) Entgegenhaltungen:
- EP-A- 0 738 890
- EP-A- 1 356 826
- CLARK M A ET AL: "LECTIN-MEDIATED MUCOSAL DELIVERY OF DRUGS AND MICROPARTICLES" ADVANCED DRUG DELIVERY REVIEWS, AMSTERDAM, NL, Bd. 43, Nr. 2/3, 30. September 2000 (2000-09-30), Seiten 207-223, XP009038338 ISSN: 0169-409X in der Anmeldung erwähnt
- BROOKING J ET AL: "TRANSPORT OF NANOPARTICLES ACROSS THE RAT NASAL MUCOSA" JOURNAL OF DRUG TARGETING, HARWOOD ACADEMIC PUBLISHERS GMBH, DE, Bd. 9, Nr. 4, 2001, Seiten 267-279, XP009038336 ISSN: 1061-186X in der Anmeldung erwähnt
- GUPTA R K ET AL: "Determination of protein loading in biodegradable polymer microspheres containing tetanus toxoid" VACCINE, BUTTERWORTH SCIENTIFIC. GUILDFORD, GB, Bd. 15, Nr. 6-7, 1. April 1997 (1997-04-01), Seiten 672-678, XP004064530 ISSN: 0264-410X in der Anmeldung erwähnt

## Beschreibung

Die vorliegende Erfindung betrifft Mikrosphären, die Antigene, insbesondere Allergene, enthalten und zur Allergietherapie dienen.

Etwa 20% der Bevölkerung leiden an IgE-vermittelten Allergien, die von leichteren Symptomen wie Heufieber bis zu schweren Episoden von Asthma oder anaphylaktischem Schock führen können. In diesen Patienten sind Mastzellen und andere Effektorzellen der Allergie mit IgE-Antikörpern beladen. Bei einem Kontakt mit dem Allergen kann es zum sogenannten Triggern der Zellen kommen, das heißt zur Freisetzung von Mediatoren wie Histamin, welches für die allergische Symptomatik verantwortlich ist.

Die gängige Standardmethode der Allergiebehandlung ist die sogenannte Hyposensibilisierung mit natürlichen Extrakten von Allergenen, welche nach langandauernder Behandlung zur Unterdrückung der Symptome führt. Der Wirkmechanismus ist dabei noch nicht völlig geklärt, jedoch beobachtet man eine Induktion von IgG Antikörpern, die als blockierende Antikörper fungieren können. Des weiteren wird nach langandauernder Therapie eine Modulation der TH Antwort diskutiert.

Um die oft gut löslichen Allergene besser immunogen zu machen, werden in der Regel sogenannte Adjuvantien verwendet, wie zum Beispiel Aluminium-Trihydroxid. Dieses adsorbiert das Allergenprotein und erhöht dadurch dessen Immunogenität.

Als eine weitere Möglichkeit zur Applikation werden in der Literatur sogenannte Mikro- oder Nanopärtikel beschrieben, die immunogene Substanzen enthalten. Beispielsweise beschreiben Johansen et al. die kontrollierte Antigenzufuhr mittels Mikrosphären aus Polymilchsäure-Polyglycolsäure-Copolymeren (sogenannte PLGA-Mikropartikel).

Die Applikation von Allergenen erfolgt meistens subkutan oder intramuskulär. Einige Literaturstellen berichten von Versuchen einer oralen Allergietherapie (European Journal of Allergy and Clinical Immunology, WHO Position Paper 44;53:20-21). Die sublinguale Immunotherapie ist bereits in klinischer Anwendung, jedoch mit moderatem Erfolg (Rakoski J, Wessner D, Int. Arch. Allergy Immunol. 2001, Nov; 126(3): 185-7).

Jedoch ist auch bereits die orale Gabe von immunogenen Substanzen, eingebunden in Mikrosphären, bekannt. So wird etwa von K.J. Maloy et al. die Induktion einer mukosalen und systemischen Immunantwort durch die orale Gabe von in PLGA-Mikropartikeln eingebundenem Ovalbumin beschrieben (K.J. Maloy et al., Immunology 1994, Apr, 81(4): 661-7). Weiterhin zeigte eine Arbeit von Pecquet et al., dass ein in PLGA-Mikrosphären eingebundenes Milchsäureallergen Mäusen zur Prophylaxe von Milchallergie oral verabreicht werden kann (Pecquel et al., Vaccine 2000, 18: 1196-1202).

Leider sind die bisherigen Behandlungsmethoden von Allergien nicht immer erfolgreich, es werden manchmal Verschlimmerungen der Symptome festgestellt, und es gibt Daten die zeigen, dass eine Hyposensibilisierung die IgE Synthese sogar anregen kann. Dies kann an der Verwendung von Aluminium-Trihydroxid (Al(OH)₃) als Adjuvans liegen, ein für viele Impfstoffe gängiges Adjuvans. Aus Tierversuchen weiß man seit langem, dass Aluminium-Trihydroxid sogar eine TH2 Antwort fördert, also eine IgE Bildung und damit eine Allergisierung bewirkt. Daraus ergibt sich die paradoxe Situation, dass allergische Patienten mit einem allergisierenden Adjuvans in Kombination mit Allergenextrakten immunisiert werden. Zusätzlich sind wiederholte Gaben von steigenden Allergenmengen erforderlich, anfangs in sogar wöchentlichen Abständen, was von Patienten häufig als lästig empfunden wird. Orale Gaben des Antigens würden dem Patienten erlauben, diese in Art einer Schluckimpfung vorteilhaft zu Hause selbst durchzuführen.

Demgemäß ist es Aufgabe der vorliegenden Erfindung, geeignete Substanzen, insbesondere Allergene, in einer Form zur Allergietherapie zur Verfügung zu stellen, die die obengenannten Nachteile bekannter Verabreichungsweisen von Allergenen vermeidet und ein zuverlässigeres Ansprechen des Patienten ermöglicht.

Der Erfindung liegt die Erkenntnis zugrunde, dass eine solche Verabreichungsform insbesondere dann eine gute Wirkung entfaltet, wenn die Mikrosphären ihre Substanzen, gezielt und über einen längeren Zeitraum an ihrem Zielgewebe freisetzen.

Gegenstand der vorliegenden Erfindung sind daher Mikrosphären zur Allergietherapie, die Antigene und/oder DNA von Antigenen, insbesondere Allergene und/oder DNA von Allergenen, enthalten, die dadurch gekennzeichnet sind, dass die Mikrosphären eine Bindungskonstante K_{B} von mindestens 1 x 10³ M⁻¹, bevorzugt mindestens 1 x 10⁴ M⁻¹ und am meisten bevorzugt mindestens 6 x 10⁴ M⁻¹, gegenüber dem spezifischen Carbohydratrest, bevorzugt alpha-L Fucose, von intestinalen und/oder nasalen Epithelzellen aufweisen.

Unter Antigene werden in dieser Erfindung nicht nur Substanzen verstanden, die vom Organismus als fremd erkannt werden und die eine Immunantwort auslösen bzw. mit einem Antikörper oder T-Zellrezeptoren reagieren, sondern auch deren Derivate. Neben den weiter unten definierten Allergenen sind insbesondere folgende Antigene bevorzugt: Virale, bakterielle, Protozoon Antigene als auch Toxine und Wurmantigene.

Es ist bevorzugt, daß nicht nur Antigene in die Mikrosphären eingeschlossen werden können sondern auch die DNA von Antigenen. Dabei besteht die Möglichkeit, Antigene oder DNA von Antigenen alleine oder gemeinsam in die Mikrosphären einzubauen. Es ist bevorzugt, daß die Antigene Allergene sind. Weiterhin ist es bevorzugt, daß die Allergene und/oder die DNA von Allergenen in die Mikrosphären einbaut werden.

Unter dem Begriff "Mikrosphären" werden dabei Partikel verstanden, die vorzugsweise kugelförmig bzw. sphärisch aufgebaut sind. Indem das bei der Produktion der Mikrosphären verwendete Lösungsmittel für das Polymer verdunstet, fallen die Mikrosphären aus und schließen dabei mitversprühte Antigene ein.

Der in der Literatur häufig verwendete Begriff der "Nanopartikel" bzw. "Nanosphären" wird somit ebenfalls unter dem Begriff "Mikrosphären" bzw. "Mikropartikel" subsumiert.

Unter dem Begriff "Allergen" werden neben natürlich vorkommenden Allergenextrakten und Allergenmolekülen auch Mutanten von Allergenen, Hypoallergene oder Teile aus Allergenmolekülen, wie etwa Peptide oder aber auch Allergenmimotope verstanden. Allergenmimotope können dabei ebenfalls Peptide sein, etwa Peptide einer Aminosäuresequenzlänge von 5 bis 25 Aminosäuren. Allergene sind hierbei in der Lage, eine Allergie, das heißt eine Überempfindlichkeitsreaktion vom Soforttyp auszulösen, die durch die Synthese von IgE Antikörpern induziert wird. Hypoallergene sind natürliche oder rekombinante Derivate eines Allergenmoleküls, welche auf Grund geringfügiger Unterschiede zur Aminosäuresequenz des Allergens eine Konformation einnehmen, bei der IgE-bindende Eigenschaften eingebüsst werden.

Insbesondere sind folgende Allergene für Mikrosphären bevorzugt: Birkenpollen (Bet v 1), Karotte (Dau c 1), Sellerie (Api g 1), Haselnuß (Cor a 1), Erlenpollen (Aln g 1) und Gräserpollen (u.a. Ph1 p 5, Phl p 1, Phl p 6, Phl p 7) sowie Haustaubmilbe (Der p 1, Der p 2) und Fische (Parvalbumin).

Insbesondere sind folgende Mimotope des Allergens Phl p 5 bevorzugt:

C S R L G R S S A W V C
C T H W Q L G E R P D C
C P S T P G E R V R H C
C R G G P D D L T A L C
C P F W V R G T T D W C
C Q V G P E C
C P S T P G S R Q N M C
C P S T P G D N P L V C
C K F V V N G R W I D C
C K F L V N G R W I D C
C R L T E N T E P L L C
C F T W G G L R D K S C
C E R A G A M E R A N C

C R S V S K E E P G M C
C K L G K F G A A R V C
C V Q D L M K S S G V C

Insbesondere ist folgendes Mimotop des Allergens Bet v 1 bevorzugt:
CRSDKDGWRLWC

In allen Fällen erfolgt eine Disulfidbrückenbildung zwischen den endständigen Cysteinen. Die Mimotope wurden mittels einer Zufalls-Peptid 10-mer Phagenbibliothek (Mazzucchelli et al., Mazzucchelli, L., Burritt, J. B., Jesaitis, A. J., Nusrat, A., Liang, T. W., Gewirtz, A. T., Schnell, F. J., and Parkos, C. A. Cell-specific peptide binding by human neutrophils. Blood, 93: 1738-1748, 1999) mittels humanem spezifischen IgE gegen Phl p 5, bzw. Bet v 1 selektioniert.

Die Struktur der Mikrosphären erlaubt es, die in den Mikrosphären enthaltenen Antigene und/oder DNA von Antigenen, insbesondere Allergene und/oder DNA von Allergenen, langsam und gleichmäßig freizusetzen. Ein Vorteil dieser stetigen Antigen-, insbesondere Allergen-Freisetzung ist es, dass keine wiederholten Gaben von steigenden Antigen-, insbesondere Allergen-Mengen wie in der konventionellen Hyposensibilisierung angewandt, verabreicht werden müssen. Das oben Ausgeführte gilt natürlich auch für die Freisetzung von DNA von Antigenen bzw. DNA von Allergenen.

Die Haftfähigkeit der Mikrosphären an mukosalem Zielgewebe wird durch deren Haftfähigkeit an Caco-2-Zellen quantifiziert.

Caco-2 Zellen (American Type Culture Catalogue Nr. HTB-37) sind intestinale Epithelzellen mit hoher Differenzierung (Poalrisiertes Wachstum, Mikrovilli, Ausbildung von Tight Junctions), ursprünglich aus einem humanen Colon Carzinom isoliert. Sie haben Oberflächeneigenschaften, die repräsentativ für das Darmepithel sind und dienen daher zum Studium der Haftfähigkeit von Mikropartikeln. Dazu werden fluoreszenzmarkierte (FITC-Cadaverin) Mikropartikel hergestellt, indem Fluorescein-Cadaverin durch die Carbodümidaktivierungsmethode covalent über die freie Carboxylgruppe des Polymers gebunden wird und diese Verbindung in der Sprühtrocknungsanlage zu Mikrosphären versprüht wird. Diese werden vorzugsweise mit einem gewünschten Lektin funktionalisiert (siehe unten) und dann mit den Caco-2 Zellen bei vorzugsweise 4°C inkubiert. Die Temperatur 4°C wird gewählt, um Internalisierung in die Epithelzellen durch Endozytosevorgänge auszuschalten und rein Bindungsfähigkeit zu beurteilen. Nach ausgiebigen Waschen mit eiskaltem Phosphatpuffer, um unspezifische Bindungen zu subtrahieren, werden die Zell-gebundenen Mikropartikel zusammen mit den Caco-2 Zellen aufgelöst und die in der Probe enthaltene Fluoreszenz in einem Fluoreszenzspektrometer bestimmt.

Bevorzugt weisen die erfindungsgemäßen Mikrosphären an ihrer Oberfläche Substanzen auf, die die Haftfähigkeit an mukosalen Zellen erhöhen und für Menschen nicht toxisch sind.

Diese sich an der Oberfläche der Mikrosphären befindlichen Substanzen dienen dabei nicht nur der verbesserten Haftung, sondern auch der Selektion des mukosalen Zielgewebes. In der Allergentherapie ist es weiterhin wünschenswert, dass die Substanzen an der Oberfläche der Mikrosphären für den Menschen nicht toxisch sind und somit keine schädlichen Nebenwirkungen hervorrufen.

Weiterhin handelt es sich bei den Substanzen an der Mikrosphärenoberfläche bevorzugt um Lektine. Dabei ist bevorzugt, daß die Lektine nicht toxisch sind. Besonders bevorzugt wird, daß die Lektine eßbar sind.

Lektine sind Proteine oder Glykoproteine, die sehr spezifisch (Poly)Saccharide auch in Lipid- oder Proteingebundener Form erkennen und binden.

Bevorzugt weisen die erfindungsgemäßen Mikrosphären zur Allergietherapie Aleuria Aurantia Lektin (AAL) an ihrer Mikrosphärenoberfläche auf.

Durch die Oberflächenmodifizierung der Mikrosphären mit Aleuria Aurantia Lektin wird die selektive Anreicherung und die Verlängerung der Verweilzeit der Mikropartikel im Darm bewirkt, wodurch eine besondere therapeutische Wirkung der Mikropartikel erzielt wird. Aleuria Aurantia Lektin (AAL) stammt aus einem eßbaren Pilz und bindet an α-L-Fucose der sogenannten M-Zellen. Diese M-Zellen leiten sich von intestinalen Epithelzellen ab, mit denen sie Oberflächeneigenschaften wie z.B. Carbohydratstrukturen (Beispiel alpha-L Fucose) teilen, und sind Bestandteil der Peyer'schen Patches, Lymphorgane des Darmes, die für die lokale Antigenaufnahme und Stimulation des Immunsystems eine Schlüsselrolle spielen. Weiterhin kann eine Art von M-Zellen in nasalen Epithel nachgewiesen und für mukosales Targeting genutzt werden (Clark et al: Adv Drug Deliv Rev 2000 Sep 30;43(2-3):207-23; oder Brookyn et al: J Drug Target 2001;9(4):267-79).

Die Isolierung und Charakterisierung von Aleuria Aurantia Lektin (AAL) wird im Buch "The Lektins: Properties, Functions and Applications in Biology and Medicine" von I. E. Liener, N. Sharon und I. J. Goldstein (Academic Press 1986) beschrieben. Das Fucose-bindende Lektin wird aus dem Fruchtkörper des Orangebecherling isoliert und wurde für das Beispiel von Vektor Laboratories (Burlingame, USA) bezogen. Es hat ein Molekulargewicht von 72,000, der isoelektrische Punkt ist zwischen 9.0-9.2, und das Lektin besteht aus 2 Untereinheiten einer einzigen Polypeptidketten aus 31 kDa. AAL besteht aus zwei gleichen Untereinheiten, die jeweils eine Bindungskonstante, K_{B}, von 6.1 x 10⁴ M⁻¹ gegenüber alpha-L Fucose aufweist.

Die Avidität (die funktionelle Affinität) kann durch multivalente Bindungen zwischen 10³ und 10⁷ mal stärker als die entsprechende Affinität einer einzelnen Bindungsstelle sein. Die Haftfähigkeit der Mikrosphären hängt somit von der Anzahl der Bindungsmöglichkeiten zwischen Mikrosphären und Epithelzellen ab, bevorzugt von der Anzahl der Lektin - Zell-Bindungen. D. h., bietet die Mikrosphäre mehr als eine Bindungsmöglichkeit an, erhöht sich automatisch auch die Bindungsstärke, da im Falle einer Ablösung sämtliche Bindungsstellen gleichzeitig von Epithelzellen gelöst werden müssten. Die Avidität K_{B} ist bevorzugt von mindestens 1x 10¹⁰ M⁻¹, noch bevorzugter von mindestens 1x 10¹¹ M⁻¹ und am meisten bevorzugt von mindestens 1x 10¹² M⁻¹ ab. Es ist des weiteren bevorzugt, daß die Avidität der Mikropartikel durch die Vielzahl von AAL auf den Mikropartikeln erreicht wird.

Die erfindungsgemäßen Mikrosphären finden Verwendung in der Allergietherapie. Sie zeichnen sich insbesondere dadurch aus, dass sie sich zielgerichtet in erhöhter Menge an mukosalen Oberflächen des gastrointestinalen und/oder nasalen Bereiches anlagern und dort ihre therapeutische Wirkung entfalten können. Daher ist die Möglichkeit der oralen oder nasalen Immunisierung gegeben. Die Vorteile einer solchen Immunisierung liegen in einer einfachen Handhabung, die auch vom Patienten bevorzugt wird. Da AAL nicht toxisch ist, ist es im Menschen unbedenklich anzuwenden und besonders bevorzugt.

Bevorzugt besitzen die Mikrosphären einen Durchmesser von 0.1-100µm, mehr bevorzugt 0.1-10µm und am meisten bevorzugt 0,2-5µm. Die Größenverteilung der Mikrosphären wurde mittels Laserdiffraktion bestimmt (Shimadzu Laser Diffraction Type Particle Size Analyzer SALD-1100). Aus der ist ersichtlich, welche Maximalgröße 50 bzw. 90% der Mikrosphären haben. Bevorzugt werden Mikrosphären, die kleiner als 10µm sind erzeugt, weil diese leichter über intestinales Epithel aufgenommen werden können. Mehr bevorzugt werden Mikrosphären, die kleiner als 8 µm sind und am meisten bevorzugt sind Mikrosphären, die kleiner sind als 5 µm.

Die Herstellung der Mikro- und/oder Nanosphären erfolgt meist durch Doppelemulsionstechnik beziehungsweise Sprühtrocknung. In einem der nachfolgenden Beispiele wird die Herstellung entsprechender Partikel exemplarisch beschrieben. Vorzugsweise werden Antigen-beladene Mikropartikeln hergestellt, indem das Antigen in der wäßrigen Lösung in ein organisches Lösungsmittel, in dem das Polymer gelöst wurde, dispergiert wird. Verschiedene Modifikationen dieses Prozesses wurden schon als Lösungsmittelverdampfungs- (S. McClean, E. Prosser, E. Meehan, D. O'Malley, N. Clarke, Z.,Ramtoola and D. Brayden, Binding and uptake of biodegradable poly-DL-lactide micro- and nanoparticles in intestinal epithelia, Eur J Pharm Sci 6 (1998) 153-163., R.K. Gupta, A.C. Chang, P. Griffin, R. Rivera, Y.Y. Guo and G.R. Siber, Determination of protein loading in biodegradable polymer microspheres containing tetanus toxoid, Vaccine 15 (1997) 672-678. ), Extraktion von Wasser-in Öl-in Wasser-Emulsionen -( J.L. Cleland, E.T. Duenas, A. Park, A. Daugherty, J. Kahn, J. Kowalski and A. Cuthbertson, Development of poly-(D,L-lactide-coglycolide) microsphere formulations containing recombinant human vascular endothelial growth factor to promote local angiogenesis, J Control Release 72 (2001) 13-24., J.L. Cleland, A. Lim, A. Daugherty, L. Barron, N. Desjardin, E.T.,Duenas, D.J. Eastman, J.C. Vennari, T., Wrin, P., Berman, K.K. Murthy and M.F. Powell, Development of a single-shot subunit vaccine for HIV-1. 5. programmable in vivo autoboost and long lasting neutralizing response, J Pharm Sci 87 (1998) 1489-1495.), Sprühtrocknungs-(N.E.S.N.W. A., Controlled release microparticles comprising core coated microparticles, US Patent, Biotek Inc., Woburn, MA, November 18, 1986.) oder Phasentrennungs-Methoden (F.J. W. Processes for preparation of microspheres, US Patent, Sandoz, Inc., E.Hanover, NJ, US, 1979.)beschrieben.

Vorzugsweise ist das Grundgerüst der Mikrosphären aus Polymeren mit funktionellen Gruppen aufgebaut. Die funktionellen Gruppen dienen insbesondere dazu, die Kraft vermittelnden Substanzen, besonders bevorzugt Lektine und am meisten bevorzugt AAL, chemisch über eine kovalente Bindung an die Oberfläche der Partikel zu binden, beispielsweise über eine Amidbindung.

Die Antigene und/oder DNA von Antigenen, bevorzugt die Allergene und/oder DNA von Allergenen können physikalisch oder chemisch in die Mikro- oder Nanosphären eingebaut werden. Vorzugsweise werden sie physikalisch eingebaut. Wie schon oben erwähnt, erfolgt die Herstellung der Mikro- und/ oder Nanosphären vorzugsweise durch Doppelemulsionstechnik bzw. Sprühtrocknung. Beim Verfahren der Sprühtrocknung wird eine Lösung des Polymers mit einem darin gelösten, dispergierten oder emulgierten Wirkstoff in einem heißen Luftstrom versprüht. Das Lösungsmittel verdunstet und verursacht eine Ausfällung des Polymers, wodurch der Wirkstoff eingebettet bzw. umhüllt wird. Die entstandenen Mikropartikel werden in einem Zyklon abgeschieden und als Pulver gewonnen. Die Polymer- und Wirkstoffkonzentration, die Sprühlösungs-förderrate, die Einlasstemperatur, die Zerstäuberluftmenge und die Aspiratorleistung können dabei als Parameter variiert werden. Je feiner die erzeugten Tropfen, desto größer ist die aktive Oberfläche und desto besser ist der Wärme- und Stoffübergang.

Bei der Erzeugung der Mikrosphären wird vorzugsweise eine Emulsion aus Ethylformiat/Wasser mit 5% Polymer und 0,2% Protein versprüht. Die Einlasstemperatur des Sprühtrockner enthält dabei vorzugsweise 45 °C und die Aspiratorleistung wird vorzugsweise auf 100% gestellt. Das Polymer ist vorzugsweise in Ehylformiat und die Proteine des Birkenpollenextrakt vorzugsweise in Wasser gelöst.

Die Funktionalisierung der Mikro- und/ oder Nanosphären verläuft vorzugsweise in zwei Schritten. Beim ersten Schritt werden die freie Carboxygruppen an der Oberfläche der Mikrosphären, vorzugsweise PLGA Mikrosphären mittels 1-Ethyl-3-(3-Dimethyl-aminopropyl)carbodiimide (EDAC) und (N-[2-Hydroxyethyl-piperazine-N'-[2-ethansulfonsäure]) (NHS) über eine Carbodiimidreaktion aktiviert. Danach erfolgt die Kopplung des Lektins unter Ausbildung einer stabilen Amidbindung.

Weiterhin bevorzugt bestehen die Mikrosphären zur Allergietherapie aus einem Grundgerüst aus einem biologisch abbaubarem Polymer oder Copolymer. Dadurch wird gewährleistet, dass die Partikel im Körper abgebaut werden können. Der Abbau muß jedoch solange hinausgezögert werden, dass die Partikel Ihren Zielort erreichen und die Antigene und/öder DNA von Antigenen, bevorzugt Allergene und/oder DNA von Allergenen kontinuierlich über einen längeren Zeitraum abgeben können. Die biologische Abbaubarkeit des Polymers oder Copolymers stellt sicher, dass sich die Mikro- beziehungsweise Nanopartikel nicht in ungewollter erhöhter Konzentration im Körper einlagern.

Besonders bevorzugt besteht das Grundgerüst der Mikrosphären zur Allergietherapie aus Polymilchsäure (PLA), welche auch unter dem Namen Polylactid bekannt ist, Polyglykolsäure (PGA), auch unter dem Namen Polyglycolid bekannt, oder aus Polymilchsäure-Polyglykolsäure-Copolymer (PGLA). Diese Polymere können im Körper langsam zu ungefährlichen Substanzen abgebaut werden. Polylaktide als Verabreichungssysteme für Peptide und Proteine werden beschrieben in Johannsen et al., European J. of Pharmaceutics and Biopharmaceutics 50 (2000): 129-146.

Denkbar als Mikrosphären-Grundgerüst wäre jedoch auch Chitosan, und zwar insbesondere für anionische Allergene.

Die Mikro- und/oder Nanosphären enthalten bevorzugt 0,1-20 Gw.-%, besonders bevorzugt 0,2-3% Gw.% Antigene und/oder DNA von Antigenen. Es ist bevorzugt, daß sich bei den Gew.%-Angaben um Angaben für Allergene und/oder DNA von Allergenen handelt.

Es ist bevorzugt, daß die Mikro- und/oder Nanosphären Mimotope von Allergenen, mehr bevorzugt Mimotope des Allergens Phl p 5 und/oder Bet v 1 enthalten. Es ist besonders bevorzugt, daß es sich um Mimotope des Allergens Phl p 5 und/oder Bet v 1 mit den Aminosäuresequenzen wie oben definiert handelt.

Weiterhin bezieht sich die vorliegende Erfindung auf ein Verfahren zur Herstellung von oben beschriebenen Mikrosphären, dadurch gekennzeichnet, dass die Mikrosphären eine Bindungskonstante K_{B} von mindestens 1 x 10⁴ M⁻¹ gegenüber dem spezifischen Carbohydratrest von intestenalen und/oder nasalen Epithelzellen aufweisen. Die Mikrosphären sind dabei wie oben beschrieben aufgebaut.

Weiterhin bezieht sich die vorliegende Erfindung auf die Verwendung von Mikrosphären zur Allergietherapie, dadurch gekennzeichnet, dass die Mikrosphären eine Bindungskonstante K_{B} von mindestens 1 x 10⁴ M⁻¹ gegenüber dem spezifischen Carbohydratrest von intestenalen und/oder nasalen Epithelzellen aufweisen. Die Mikrosphären besitzen dabei einen Aufbau wie oben beschrieben. Dabei ist die Avidität K_{B} ist vorzugsweise mindestens 10¹⁰ M⁻¹, noch mehr bevorzugt 1 x 10¹¹ M¹ und am meisten bevorzugt 1 x 10¹² M⁻¹.

Nachstehend ist eine Ausführungsform der erfindungsgemäßen Mikrosphären anhand der Figuren beispielhaft näher erläutert. Dabei zeigen:
Figur 1: IgG Titer gegen Bet v 1 nach 2-maliger orale Immunisierung mit Mikropartikeln; aufgetragen ist die IgG-Detektion gegen Bet v 1. ELISA-Platten wurden mit rekombinantem Bet v 1 beschichtet, mit Mausseren (1:100 verdünnt) inkubiert, und gebundenes IgG durch einen Peroxidase-markierten Anti-Maus IgG detektiert. Die Reaktion wurde durch Substratzugabe visualisiert und die Reaktivität mit einem ELISA Reader gemessen. Die Werte sind direkt proportional der Menge des gebundenen Antikörpers.
   Die Abkürzungen bedeuten: MS = Mikrosphären, BP = Birkenpollenextrakt, AAL = Aleuria Aurantia Lektin, WGA = Weizenkeim Agglutinin, PIS = Präimmunserum, 1.MIS = erstes Immunserum, 2.MIS = zweites Immunserum, OD = Optische Dichte
Figur 2: Überprüfung der Antigenität von in Mikrosphären verpacktem Birkenpollenextrakt nach gastrischem Verdau; die graphische Darstellung zeigt die Antigenität der verschiedenen Mikropartikel in zeitlicher Abhängigkeit gastrischer Verdauung. Die Abkürzungen haben folgende Bedeutung:
   BP: Birkenpollenextrakt, MS: Mikrosphären, AAL: Aleuria Aurantia Lektin, WGA: Weizenkeim Agglutinin.
Figur 3: Qualitative Überprüfung der Haftfähigkeit unterschiedlicher Mikrosphärenpräparationen an humanen intestinalen Epithelzellen in der Immunofluoreszenz.
Figur 4: Quantitative Überprüfung der Haftfähigkeit unterschiedlicher Mikrosphärenpräparationen an humanen intestinalen Epithelzellen im Fluoreszenz-ELISA.

Aleuria Aurantia Lektin (AAL) besitzt den Vorteil gegenüber vielen Lektinen, nicht toxisch zu sein. Ein weiteres, allerdings toxisches Lektin ist Weizenkeim Agglutinin (WGA), von dem vermutet wird, dass es aufgrund seiner Glykan-bindenden Eigenschaften an intestinale Epitelzellen bindet, und zwar insbesondere durch Bindung an N-Acetylglykosamin.

Wie in den unten beschriebenen Beispielen gezeigt wird, besitzt AAL jedoch den weiteren und unerwarteten Vorteil gegenüber WGA, signifikant mehr Antigen an die mukosale Oberfläche zu binden. Dies mag an einer verbesserten Haftung liegen, wird aber auf jeden Fall als Vorteil angesehen, da diese Eigenschaft letztlich dafür sorgt, dass die in die AALoberflächenmodifizierten Mikropartikel eingebauten Allergene zielgerichtet und in erhöhter Menge an ihren therapeutischen Einsatzort gelangen können. Außerdem erübrigt sich dabei die wiederholte Gabe von steigenden Allergenmengen in kurzen Abständen, weil die Mikrosphären in der Lage sind, die Allergene langsam und stetig in das Zielgewebe freizusetzen.

### Beispiele:

### Beispiel 1: Allergenhaltige, Lektin-gekoppelte PLGA-Mikrosphären für die orale Allergie-Prophylaxe und Therapie.

PLGA-Mikrosphären (MS) wurden mittels Sprühtrocknung gewonnen. Bei diesem Verfahren wurde eine Emulsion aus Wasser und Ethylformiat mit Birkenpollenextrakt in einem heißen Luftstrom versprüht. Der Median der Mikrosphären wurde mittels Laser-Diffraktion ermittelt und betrug 5,5 µm. Die Proteinmenge der Mikropartikel wurde mit 40 ug/mg Mikropartikel bestimmt. Die Funktionalisierung der Mikrosphären mit den jeweiligen Lektinen erfolgte unter Ausbildung einer Amidbindung. Bei der Erzeugung der Mikrosphären wird eine Emulsion aus Ethylformiat/Wasser mit 5% (w/v) Polymer und 0,2% (w/v) Protein versprüht. Das Polymer wird dabei im Ethylformiat gelöst, die Proteine des Birkenpollenextraktes in Wasser. Die Emulsion wird in einem heißen Luftstrom versprüht. Das Lösungsmittel verdunstet und verursacht eine Ausfällung des Polymers, wodurch die Proteine des Birkenpollenextraktes eingebettet werden. Für die Proteinbestimmung wurden dann die Mikrosphären in 0,05N NaOH/1%SDS gelöst und mittels eines Färbetest (Bichinchoninsäure-Assay) quantifiziert.

Die Funktionalisierung der mit Proteinen des Birkenpollenextraktes gefüllten Mikro- und/ oder Nanosphären verläuft in zwei Schritten. Dazu werden die Partikel in Hepes-Puffer pH 7,2 aufgenommen und durch Zugabe von EDAC/NHS über eine Carbodiimidreaktion aktiviert. AAL wird zugeben und das Lektin über eine Ausbildung einer stabilen Amidbindung an die Mikro- und/ oder Nanosphären gekoppelt. Die restlichen aktivierten Carboxygruppen werden mit Glycin abgesättigt.

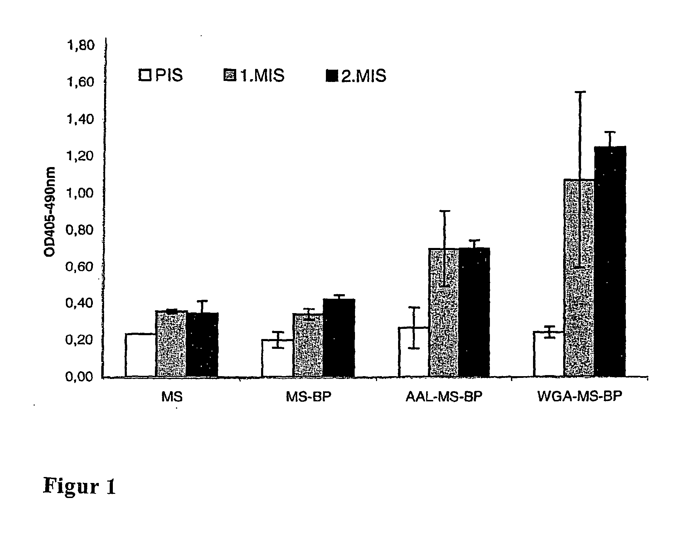

Die Ergebnisse des Immunisierungsmodells sind in Figur 1 gezeigt. 4 Gruppen von je 5 Balb/c Mäusen wurden mit Mikropartikeln zweimal (Tag 0 und Tag 21) an jeweils drei aufeinanderfolgenden Tagen mit Mikropartikeln in PBS (Volumen 150 microL) intragastral immunisiert. Die Mikropartikel waren entweder leer oder mit Birkenpollenproteinen bepackt. Ein Teil der Mikropartikel, die mit Birkenpollenproteinen bepackt waren, war außerdem zusätzlich mit Aleuria Aurantia Lektin oder Weizenkeim Agglutinin funktionalisiert. Vor und nach zwei Wochen nach den Immunisierungen wurde den Mäusen Blut abgenommen und auf das Vorliegen von spezifischen Anti-Bet v 1 Antikörpern untersucht. Dies geschah in einem ELISA. Dabei wurde Birkenpollenextrakt in einer Konzentration von 10 micorg/ml Carbonatpuffer, pH 9,6 beschichtet. Nach Blockierung mit PBS/0,1% BSA wurden Maussera (1:100 in Blockierungspuffer verdünnt) inkubiert. Nach Waschen mit PBS wurde gebundenes IgG mit anti-Maus Antikörpern, Peroxidase konjugiert, detektiert. Die Reaktion wurde durch Substrazzugabe entwickelt und in einem ELISA Reader erfolgte die Auswertung durch Ablesung bei OD 405-490 nm.

Wie in Figur 1 zu sehen ist, zeigten die Gruppen, die mit leeren beziehungsweise mit Birkenpollenproteinen bepackten Mikropartikeln gefüttert wurden, im ELISA weder im ersten Immunserum (1. MIS-grauer Balken) noch im zweiten Immunserum (2. MIS-grau-schwarzer Balken) einen relevanten Anstieg an Bet v 1 spezifischen IgG-Antikörpern gegenüber dem Präimmunserum. Hingegen zeigten beide Gruppen, bei denen die bepackten Mikropartikel zusätzlich außen noch mit Lektinen funktionalisiert wurden, einen deutlichen Anstieg der gegen Bet v 1 gerichteten IgG-Antikörper.

### Beispiel 2: Gastrische Verdauung.

Um die Antigenität von mikrosphären-verpacktem Birkenpollenextrakt (BP-MS) nach gastrischem Verdau zu überprüfen, wurden Mikrosphären mit Pepsin bei pH 1,5 für unterschiedliche Zeitintervalle inkubiert. In mikrosphären-verpacktes Birkenpollenprotein konnte auch noch nach zwei Stunden Verdau antigenetisch detektiert werden, hingegen wurde nicht verpacktes Birkenpollenprotein innerhalb von Sekunden zerstört. AALgekoppelte Mikropartikel reagierten in diesem Versuch genauso vorteilhaft wie andere Mikrosphären-Präparationen (MS: Mikrosphären allein, MS-WGA: mit Weizenkeim Agglutinin gekoppelte Mikrosphären) und schützten das Protein vor dem Verdau. Weiterhin zeigt dieses Beispiel, dass Birkenpollenprotein beim Einpacken in Mikrosphären nichts an Antigenität gegenüber unbehandelten Extrakten verliert.

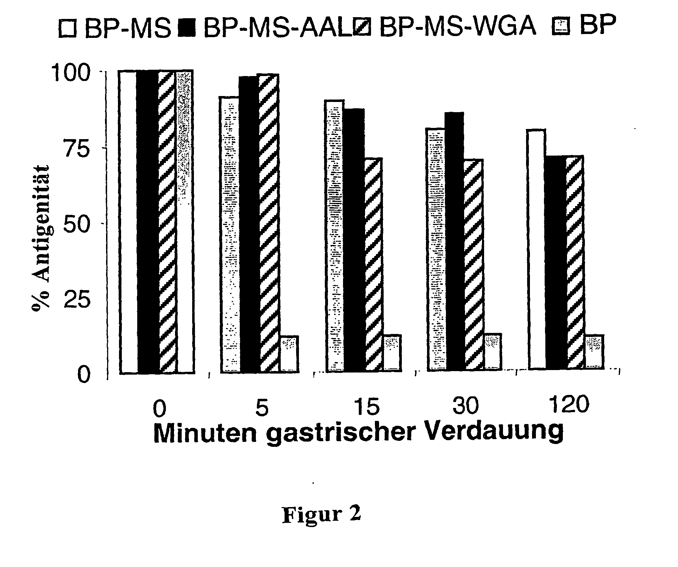

Die Auswertung des Versuchs erfolgte in einem ELISA: das Birkenpollenprotein wurde aus Mikrosphären extrahiert und die Lösung zur Beschichtung von ELISA-Platten verwendet. Gebundenes Birkenpollen-Hauptallergen Bet v 1 wurde mit einem polyklonalen Kaninchen Anti-Bet v 1-Antikörper und dann mittels eines zweiten Peroxidase markierten Anti-Kaninchen IgG-Antikörpers detektiert. Die Reaktion wurde durch Substratzugabe visualisiert und das Signal in einem ELISA Reader gemessen. Das gebundene Antigen ist der Reaktivität und daher dem Signal direkt proportional. Die Ergebnisse sind in Figur 2 graphisch dargestellt.

### Beispiel 3: Qualitative Haftfähigkeit unterschiedlicher Mikrosphären-Präparationen.

Die qualitative Überprüfung der Haftfähigkeit unterschiedlicher Mikrosphären-Präparationen an humanen intestinalen Epithelzellen erfolgte mit der Immunofluoreszenz-Methode. Die Zelllinie Caco-2 wurde auf Glasplättchen ausgesät und bei 4 °C inkubiert mit:
1. Birkenpollenextrakt verpackt in Mikrosphären (BP-MS), oder
2. Birkenpollenextrakt verpackt in Mikrosphären, gekoppelt mit Weizenkeim Agglutinin (BP-WGA-MS), oder
3. Birkenpollenextrakt verpackt in Mikrosphären, gekoppelt mit Aleuria Aurantia Lektin (BP-AAL-MS).

Zum Sichtbarmachen wurden die Mikrosphären mit dem Fluoreszenz-Farbstoff Fluorescein-Isothiocyanat(FITC)-Cadaverin beladen. Die Visualisierung der Epithelzellen erfolgte mit einem Kaninchen anti-PLAP Antikörper, gefolgt von ALEXA 568-konjugiertem Anti-Kaninchen IgG. Die Zellkerne wurden mit Hoechst Dye gefärbt.

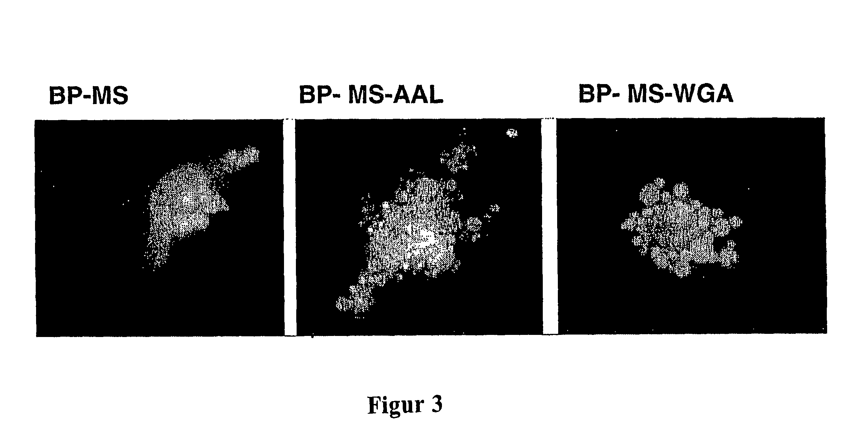

Gegenüber nicht-funktionalisierten Mikrosphären wird in diesem Versuch deutlich, dass Lektin-Funktionalisierung die Anbindung der Partikel an das Epithel signifikant verbessert. Die Ergebnisse sind in Figur 3 gezeigt.

Beispiel 4: Quantitative Überprüfung der Haftfähigkeit unterschiedlicher Mikrosphären-Präparationen.

Die quantitative Überprüfung der Haftfähigkeit unterschiedlicher Mikrosphären-Präparationen an humane intestinalen Epithelzellen erfolgte im Fuoreszenz-ELISA. Die Zelllinie Caco-2 wurde in 96-well Gewebekulturplatten gezüchtet, bis sie geschlossene einschichtige Lagen bildete. Dann erfolgte eine Inkubation bei 4 °C mit:
1. Birkenpollenextrakt verpackt in Mikrosphären (BP-MS), oder
2. Birkenpollenextrakt verpackt in Mikrosphären, gekoppelt mit Weizenkeim Agglutinin (BP-WGA-MS), oder
3. Birkenpollenextrakt verpackt in Mikrosphären, gekoppelt mit Aleuria Aurantia Lektin (BP-AAL-MS).

Zur Sichtbarmachung wurden mit FITC-Cadaverin markierte Mikrosphären verwendet. Die Visualisierung der Epithelzellen erfolgte durch Fluoreszenz-Detektion bei 485/535 nm. Das Signal ist der Anzahl gebundener Partikel direkt proportional.

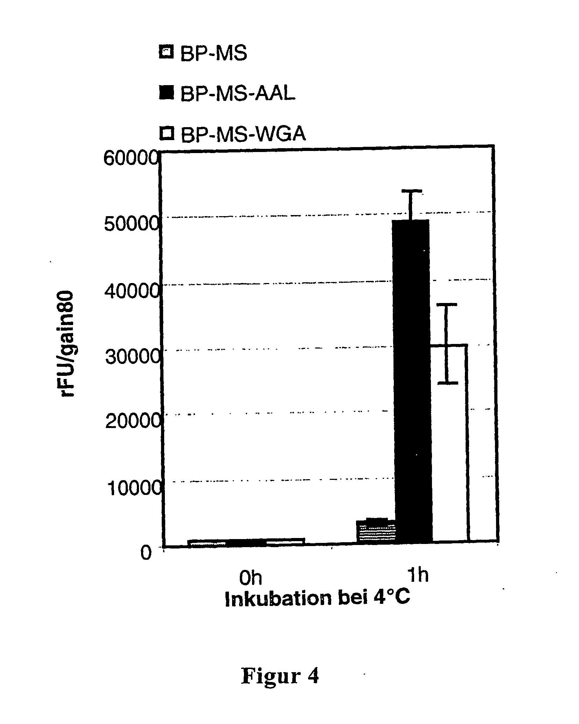

Gegenüber nicht-funktionalisierten Mikrosphären wird in diesem Versuch deutlich, dass Lektin-Funktionalisierung die Anbindung der Partikel an das Epithel signifikant verbessert. Vor allem aber besitzt AAL eine deutlich erhöhte Haftfähigkeit gegenüber WGA. Die Ergebnisse sind in Figur 4 dargestellt.

## Patentansprüche

1. Mikrosphären zur Allergietherapie, die Antigene und/oder DNA von Antigenen enthalten, **dadurch gekennzeichnet, dass** die Mikrosphären eine Bindungskonstante K_{B} von mindestens 1 x 10⁴ M⁻¹ gegenüber dem spezifischen Carbohydratrest von intestinalen und/oder nasalen Epithelzellen aufweisen.

2. Mikrosphären zur Allergietherapie nach Anspruch 1, **dadurch gekennzeichnet, daß** die Mikrosphären eine Avidität K_{B} von mindestens 1 x 10¹⁰ M⁻¹ gegenüber dem spezifischen Carbohydratrest von intestinalen und/oder nasalen Epithelzellen aufweisen.

3. Mikrosphären zur Allergietherapie nach Anspruch 1 oder 2, **dadurch gekennzeichnet, dass** die Mikrosphären an ihrer Oberfläche zektine aufweisen, die die Haftfähigkeit an mukosalen Zellen erhöhen.

4. Mikrosphären zur Allergietherapie nach einem der vorstehenden Ansprüche, **dadurch gekennzeichnet, dass** der spezifische Carbohydratrest α-L Fucose ist.

5. Mikrosphären zur Allergietherapie nach Anspruch 4, **dadurch gekennzeichnet, dass** die Substanz an der Mikrosphärenoberfläche ein nicht toxisches Lektin ist.

6. Mikrosphären zur Allergietherapie nach Anspruch 4 oder 5, **dadurch gekennzeichnet, dass** Lektin eßbar ist.

7. Mikrosphären zur Allergietherapie nach Anspruch 4-6, **dadurch gekennzeichnet, dass** das Lektin Aleuria-Aurentia-Lektin ist.

8. Mikrosphären zur Allergietherapie nach einem der vorstehenden Ansprüche, **dadurch gekennzeichnet, dass** die Mikrosphären einen Durchmesser von 0,1 bis 100 µm haben.

9. Mikrosphären zur Allergietherapie nach einem der vorstehenden Ansprüche, **dadurch gekennzeichnet, dass** das Grundgerüst der Mikrosphären aus Polymeren besteht.

10. Mikrosphären zur Allergietherapie nach Anspruch 9, **dadurch gekennzeichnet, dass** das Grundgerüst der Mikrosphären aus Polymeren mit funktionellen Gruppen besteht.

11. Mikrosphären zur Allergietherapie nach einem der Ansprüche 9 oder 11, **dadurch gekennzeichnet, dass** das Grundgerüst der Mikrosphären aus biologisch abbaubaren Polymeren oder Copolymeren besteht.

12. Mikrosphären zur Allergietherapie nach einem der Ansprüche 8-11, **dadurch gekennzeichnet, dass** das Grundgerüst der Mikrosphären aus Polymilchsäure, Polyglycolsäure oder aus Polymilchsäure-Polyglycolsäure-Copolymer besteht.

13. Mikrosphären zur Allergietherapie nach einem der Ansprüche 9 bis 12, **dadurch gekennzeichnet, dass** das Aleuria Aurantia Lektin über eine kovalente Bindung an die Polymere gebunden ist.

14. Mikrosphären zur Allergietherapie nach einem der vorstehenden Ansprüche, **dadurch gekennzeichnet, dass** die Mikrosphären 0,1-20 Gw.-% Antigene und/oder DNA von Antigenen enthalten.

15. Mikrosphären zur Allergietherapie nach einem der vorhergehenden Ansprüche, **dadurch gekennzeichnet, daß** die Antigene und/oder DNA von Antigenen Allergene und/oder DNA von Allergenen sind.

16. Mikrosphären zur Allergietherapie nach einem der vorhergehenden Ansprüche, **dadurch gekennzeichnet, daß** die Antigene Mimotope des Allergens Phl p 5 und/oder des Allergens Bet v 1 sind.

17. Verfahren zur Herstellung von Mikrosphären nach einem der vorstehenden Ansprüche, **dadurch gekennzeichnet, dass** die Mikrosphären zunächst mir Antigenen und/oder DNA von Antigenen beladen werden und anschließend die Mikrosphären funktionalisiert werden.

18. Verwendung von Mikrosphären nach einem der vorstehenden Ansprüche 1-17 zur Herstelling mies Aizneimittels für die Allergietherapie.

## Claims

1. Microspheres for allergy therapy containing antigens and/or DNA of antigens, **characterized in that** the microspheres have a binding constant K_{B} of at least 1 x 10⁴ M⁻¹ toward the specific carbohydrate residue of intestinal and/or nasal epithelial cells.

2. Microspheres for allergy therapy according to claim 1, **characterized in that** the microspheres have an avidity K_{B} of at least 1 x 10¹⁰ M⁻¹ toward the specific carbohydrate residue of intestinal and/or nasal epithelial cells.

3. Microspheres for allergy therapy according to claim 1 or 2, **characterized in that** the microspheres have lectins on their surface which increase the adhesion to mucosal cells.

4. Microspheres for allergy therapy according to any of the above claims, **characterized in that** the specific carbohydrate residue is alpha-L-fucose.

5. Microspheres for allergy therapy according to claim 4, **characterized in that** the substance on the microsphere surface is a nontoxic lectin.

6. Microspheres for allergy therapy according to claims 4 or 5, **characterized in that** lectin is edible.

7. Microspheres for allergy therapy according to claims 4-6, **characterized in that** the lectin is Aleuria aurantia lectin.

8. Microspheres for allergy therapy according to any of the above claims, **characterized in that** the microspheres have a diameter of from 0.1 to 100 µm.

9. Microspheres for allergy therapy according to any of the above claims, **characterized in that** the skeleton of the microspheres consists of polymers.

10. Microspheres for allergy therapy according to claim 9, **characterized in that** the skeleton of the microspheres consists of polymers with functional groups.

11. Microspheres for allergy therapy according to either of claims 9 and 10, **characterized in that** the skeleton of the microspheres consists of biodegradable polymers or copolymers.

12. Microspheres for allergy therapy according to any of claims 8-11, **characterized in that** the skeleton of the microspheres consists of polylactic acid, polyglycolic acid or of poly(lactic-co-glycolic acid) copolymer.

13. Microspheres for allergy therapy according to any of claims 9 to 12, **characterized in that** the Aleuria aurantia lectin is bound to the polymers by a covalent bond.

14. Microspheres for allergy therapy according to any of the above claims, **characterized in that** the microspheres contain 0.1-20 wt.% of antigens and/or DNA of antigens.

15. Microspheres for allergy therapy according to any of the above claims, **characterized in that** the antigens and/or DNA of antigens are allergens and/or DNA of allergens.

16. Microspheres for allergy therapy according to any of the above claims, **characterized in that** the antigens are mimotopes of the allergen Phl p 5 and/or of the allergen Bet v 1.

17. A method for producing microspheres according to any of the above claims, **characterized in that** the microspheres are first loaded with antigens and/or DNA of antigens, and the microspheres are then functionalized.

18. Use of microspheres according to any of the above claims 1-17 for producing a medicine for allergy therapy.

## Revendications

1. Microsphères pour le traitement des allergies, contenant des antigènes et/ou des ADN d'antigènes, **caractérisées en ce que** les microsphères présentent une constante de liaison K_{B} d'au moins 1 x 10⁴ M⁻¹ vis-à-vis du radical d'hydrate de carbone spécifique de cellules épithéliales intestinales et/ou nasales.

2. Microsphères pour le traitement des allergies selon la revendication 1, **caractérisées en ce que** les microsphères présentent une avidité K_{B} d'au moins 1 x 10¹⁰ M⁻¹ vis-à-vis du radical d'hydrate de carbone spécifique de cellules épithéliales intestinales et/ou nasales.

3. Microsphères pour le traitement des allergies selon la revendication 1 ou 2, **caractérisées en ce que** les microsphères comprennent au niveau de leur surface des lectines qui augmentent l'adhésion aux cellules des muqueuses.

4. Microsphères pour le traitement des allergies selon l'une quelconque des revendications précédentes, **caractérisées en ce que** le radical d'hydrate de carbone spécifique est l'α-L-fucose.

5. Microsphères pour le traitement des allergies selon la revendication 4, **caractérisées en ce que** la substance au niveau de la surface des microsphères est une lectine non toxique.

6. Microsphères pour le traitement des allergies selon la revendication 4 ou 5, **caractérisées en ce que** la lectine est comestible.

7. Microsphères pour le traitement des allergies selon les revendications 4 à 6, **caractérisées en ce que** la lectine est la lectine d'Aleuria aurantia.

8. Microsphères pour le traitement des allergies selon l'une quelconque des revendications précédentes, **caractérisées en ce que** les microsphères ont un diamètre de 0,1 à 100 µm.

9. Microsphères pour le traitement des allergies selon l'une quelconque des revendications précédentes, **caractérisées en ce que** le squelette de base des microsphères consiste en des polymères.

10. Microsphères pour le traitement des allergies selon la revendication 9, **caractérisées en ce que** le squelette de base des microsphères consiste en des polymères dotés de groupes fonctionnels.

11. Microsphères pour le traitement des allergies selon l'une quelconque des revendications 9 ou 11, **caractérisées en ce que** le squelette de base des microsphères consiste en des polymères ou copolymères biodégradables.

12. Microsphères pour le traitement des allergies selon l'une quelconque des revendications 8 à 11, **caractérisées en ce que** le squelette de base des microsphères consiste en un acide polylactique, un acide polyglycolique ou un copolymère d'acide polylactique-acide polyglycolique.

13. Microsphères pour le traitement des allergies selon l'une quelconque des revendications 9 à 12, **caractérisées en ce que** la lectine d'Aleuria aurantia est reliée aux polymères par une liaison covalente.

14. Microsphères pour le traitement des allergies selon l'une quelconque des revendications précédentes, **caractérisées en ce que** les microsphères contiennent 0,1 à 20 % en poids d'antigènes et/ou d'ADN d'antigènes.

15. Microsphères pour le traitement des allergies selon l'une quelconque des revendications précédentes, **caractérisées en ce que** les antigènes et/ou ADN d'antigènes sont des allergènes et/ou des ADN d'allergènes.

16. Microsphères pour le traitement des allergies selon l'une quelconque des revendications précédentes, **caractérisées en ce que** les antigènes sont des mimotopes de l'allergène Phl p 5 et/ou de l'allergène Bet v 1.

17. Procédé de fabrication de microsphères selon l'une quelconque des revendications précédentes, **caractérisé en ce que** les microsphères sont tout d'abord chargées avec des antigènes et/ou des ADN d'antigènes, puis les microsphères sont fonctionnalisées.

18. Utilisation de microsphères selon l'une quelconque des revendications 1 à 17 précédentes pour la fabrication d'un médicament pour le traitement des allergies.
